# EUROPEAN PATENT SPECIFICATION

(11) **EP 0 192 033 B1**
(45) Date of publication and mention of the grant of the patent: **25.09.1996**
(21) Application number: 86100279.8
(22) Date of filing: 10.01.1986
(51) Int. Cl.: C12N 15/31, C12P 21/02, A61K 39/118, C12Q 1/68, G01N 33/569

(54) **Chlamydia major outer membrane protein**
Hauptprotein der Aussenmembran von Chlamydia
Protéine principale de la membrane externe de Chlamydia

(30) Priority: 14.01.1985 US 692001
(43) Date of publication of application: 27.08.1986
(73) Proprietor: CHIRON CORPORATION, Emeryville, California 94608 (US); WASHINGTON RESEARCH FOUNDATION, Seattle, WA 98105-6099 (US)
(72) Inventor: Agabian, Nina, San Francisco California 94131 (US); Stephens, Richard, Seattle Washington 98199 (US); Kuo, Cho-Chou, Washington 98115 (US); Mullenbach, Guy T., California 94618 (US)
(74) Representative: Glawe, Delfs, Moll & Partner

(56) References cited:
- EP-A- 0 059 624
- EP-A- 0 124 124
- INFECTION AND IMMUNITY, vol. 45, no. 3, September 1984, pages 637-641, American Society for Microbiology; I. ALLAN et al.: "Molecular cloning of the major outer membrane protein of Chlamydia trachomatis"
- PROC. NATL. ACAD. SCI. USA, vol. 80, March 1983, pages 1194-1198; R.A. YOUNG et al.: "Efficient isolation of genes by using antibody probes"
- CHEMICAL ABSTRACTS, vol. 103, 1985, page 151, abstract no. 1544n, Columbus, Ohio, US; R. KAUL et al.: "Cloning and expression in Escherichia coli of a species-specific Chlamydia trachomatis outer membrane antigen", & FEMS MICROBIOL. LETT. 1985, 27(1), 7-12
- INFECTION AND IMMUNITY, vol. 47, no. 3, March 1985, pages 713-718, American Society of Microbiology; R.S. STEPHENS et al.: "Molecular cloning and expression of Chlamydia trachomatis major outer membrane protein antigens in Escherichia coli"
- BIOLOGICAL ABSTRACTS, vol. 80, 1985, abstract no. 39611; F.E. NANO et al.: "Partial amino acid sequence and molecular cloning of the encoding gene for the major outer membrane protein of Chlamydia trachomatis", & INFECT IMMUN 48(2), 372-377, 1985

## Description

Chlamydia trachomatis is a major human pathogen responsible for such diseases as trachoma, inclusion conjunctivitis, pneumonia, lymphogranuloma venereum, and mucous membrane genital tract infections such as cervicitis and urethritis. The latter infections may develop systemic complications resulting in epididymitis, salpingitis, or perihepatitis. Thus, it would be of great medical interest to develop reagents and vaccines useful in the diagnosis and treatment of patients infected with Chlamydia trachomatis.

Chlamydia trachomatis species are divided into two biovars, the trachoma biovar and the lymphogranuloma venereum (LGV) biovar, based on the disease inducing characteristics of the species. Each biovar, in turn, includes a number of serovars based on specific serological determinants. The trachoma biovar contains twelve known serovars, while the LGV biovar includes three known serovars. Unique serological determinants which are characteristic of the species, biovar, and serovar have been associated with the major outer membrane protein (MOMP), which protein accounts for over 60% of the total cell wall protein synthesized during chlamydial development. The major outer membrane protein of each serovar appears to have a unique structure and includes species-specific, biovar-specific, and serovar-specific epitopes, allowing Chlamydia trachomatis to be classified by reaction with a panel of monoclonal antibodies specific for the various epitopes. The molecular weight of the various MOMP's generally ranges from about 38kD to 45kD. The serovars display varying antigenic complexity, with certain serovars eliciting broad cross-reactivity with others in the same biovar, while other serovars display little or no such cross-reactivity.

Vaccines utilizing purified and unpurified preparations of intact Chlamydia trachomatis have been prepared and tested on monkeys. While successful protection against subsequent challenge with the same chlamydial serovar was achieved, it was found that heterologous serovar challenge resulted in more severe pathology than that experienced by controls who had not been immunized. In human trials, immunization with the vaccines afforded significant protection against the serovar of the vaccine for up to two years, but hypersensitivity resulted from infection with heterologous serovars.

### 2. Description of the Relevant Art

The nature of the major outer membrane protein and its relation to the biovars and serovars of Chlamydia trachomatis are discussed in Grayston and Wang (1975) J. Infect. Dis. 132:87-105; Stephens et al. (1982) J. Immunol. 128:1083-1089; and Caldwell et al. (1981) Infect. Immun. 31:1161-1176. Inhibition of infectivity of Chlamydia trachomatis by both anti-chlamydial antisera and monoclonal antibodies has been demonstrated. Caldwell and Perry (1982) Infect. Immun. 38:745-754; and Clark et al. (1982) Infect. Immun. 38:1273-1278. Vaccine trials conducted with intact chlamydial elementary bodies are reported by Collier (1961) Lancet 1:795-800; Wang et al. (1967) Amer. J. Ophthal. 63:1615-1630; and Woolridge et al. (1967) Amer. J. Ophthal. 63:1645-1653. The cloning and expression of a gene encoding a 74,000 dalton chlamydial antigen in E. coli is reported by Stephens et al. (1983) Abstracts Annual Meeting American Society of Microbiology, B29, p. 35. Stephens et al. failed to obtain expression of a major outer membrane protein. Wenman and Lovett (1982) Nature 296:68-70, report the expression of a 19,000 dalton Chlamydia trachomatis polypeptide. The polypeptide does not appear to be involved in the major outer membrane protein. Allan et al. (1984) Infect. Immun. 45:637-641, recently reported the cloning of the major outer membrane protein gene. Nano et al. (1985) Infec. Immun. 48:372-377 report the sequencing of the first 25 N-terminal amino acids of the major outer membrane protein and the cloning of at least a portion of the gene. An immunoassay for the detection of Chlamydia trachomatis antigen is described in U.S. Patent No. 4,497,899.

### SUMMARY OF THE INVENTION

Polypeptide compositions having immunological activity corresponding to that of a major outer membrane protein (MOMP) of Chlamydia trachomatis are produced by expressing a chimeric DNA construct comprising a MOMP polynucleotide at least Z7 base pairs in length from the sequence of Appendix A under the regulatory control of a regulatory system recognized by a unicellular expression host. The MOMP polynucleotide may code for the entire protein or for a fragment thereof, and may be expressed in conjunction with another structural gene to yield a fused translation product. Polypeptide compositions according to the invention comprise from nine to fifty amino acids having a sequence coming within the amino acid sequence of Appendix A and may be characterized by the presence of non-interfering amounts of substances derived from the expression, which presence may be used to distinguish the polypeptides of the present invention from the natural polypeptides. The polypeptide compositions of the present invention are useful as substitutes for the naturally-occurring MOMP's of Chlamydia trachomatis, particularly as immunological reagents, e.g., in serological assays to detect the presence of antibodies in blood, the immunogenic substance in vaccines, and the like. The MOMP polynucleotides will also be useful as labelled probes for diagnostics.

### DESCRIPTION OF THE SPECIFIC EMBODIMENTS

Methods and compositions as defined in the claims are provided for the efficient expression of polypeptides demonstrating immunological activity analogous to that of a major outer membrane protein (MOMP) of Chlamydia trachomatis. By analogous immunological activity, it is meant that, when administered to a vertebrate, the polypeptides will elicit an immunological response which is cross-reactive with antibodies elicited by administration of the natural MOMP. The present invention employs a DNA construct includinq a MOMP polynucleotide or fragment thereof at least Z7 based pairs in length from the sequence of Appendix A encoding a polypeptide under the transcriptional and translational control of suitable regulatory sequences. MOMP polypeptides free from other chlamydial antigens are obtained by expressing the MOMP polynuclectides in a unicellular host other than Chlamydia trachomatis.

The MOMP polynucleotides of the present invention may be initially derived from any of the chlamydial serovars and may be employed in a natural or modified form. MOMP nucleotides having a sequence corresponding to an intact natural MOMP gene will usually be employed when it is desired to express the entire MOMP, although it will be possible to alter the sequence for a desired purpose, e.g., to conform to the codon bias of the expression host (as discussed below), or to create or delete restriction sites, so long as the amino acid sequence is not significantly altered. Shorter DNA sequences corresponding to portions of the MOMP gene will be employed when it is desired to produce only a fragment of the natural proteins. Usually, such sequences will encode for an epitopic region(s), comprising at least 27 bp, usually at least 36 bp, preferably at least 45 bp, and may be much longer.

It will sometimes be desirable to express the MOMP polynucleotide together with other gene(s) in order to provide fused translation products having desirable properties. For example, when producing low molecular weight MOMP polypeptide fragments (below about 5kD), it may be desirable to fuse the MOMP polypeptide to an immunogenic carrier, e.g., tetanus toxoid or hepatitis B surface antigen. It is also possible to fuse MOMP fragments from more than one serovar to each other and/or a gene expressing a suitable immunogenic carrier. Conveniently, the MOMP polynucleotide will be inserted in proper reading frame with the fused gene and under the regulatory control of the regulatory system of the fused gene. Recovery of the gene product may be facilitated by employing a secretory protein as the fused gene product, as described below.

The MOMP polypeptides may be glycosylated, partially glycosylated, or unglycosylated, depending on the nature of the expression host. Generally, prokaryotes such as E. coli will provide no glycosylation of the translated MOMP gene products, while yeast and mammalian cell culture will provide partial or substantial glycosylation. Thus, it will be possible to vary the final MOMP product by appropriate selection of the expression host.

The MOMP polynucleotide may be synthetic or natural, or combinations thereof. A natural MOMP gene (or a portion thereof) may be obtained by preparing a Chlamydia trachomatis genomic library and screening for the presence of the MOMP gene. Screening may be accomplished using antibodies for the gene product or using labelled DNA probes specific for the polynucleotide. Both methods are exemplifed in the Experimental section hereinafter. Suitable antibodies are commercially available or may be prepared from purified MOMP obtained from Chlamydia trachomatis by well known techniques. Suitable DNA probes may be obtained based on the amino acid sequence of the MOMP, or based on the polynucleotide sequence which is reported hereinafter for the MOMP of the L₂ serovar (see Appendix B). Conveniently, the λgt11/L2/33 clone which has been deposited in connection with this patent application may be labelled and used as a screening probe. A specific method for selecting a clone expressing the MOMP of the L₂ serovar from a Chlamydia trachomatis genomic library is set forth in the Experimental section hereinafter. This method can be modified to allow for selection of MOMP gene(s) from other serovars.

Synthetic polynucleotide sequences encoding for at least a portion of the MOMP gene of Chlamydia trachomatis may also find use, either alone or in combination with the naturally-occurring sequences. Coding for the synthetic sequences may be based on either the reported amino acid sequences for the MOMP's or on the polynucleotide sequences which are determined from the MOMP genes by known techniques. When used for preparing polypeptides as immunological reagents or as vaccines, it is usually desirable that the synthetic nucleotide fragment code for an oligopeptide corresponding to an epitopic site of the natural MOMP. Often, such epitopic sites may be inferred from the folding rules of Chou and Fasman (1974) Biochemistry 13:211-222, in conjunction with an analysis of hydrophobic and hydrophilic regions of the protein as taught by Hopp and Woods (1981) Proc. Natl. Acad. Sci. USA 78:3824-3828. Alternatively, the DNA sequences encoding the polypeptide regions which react with particular monoclonal antibodies may be identified by the high-density phage procedure described by Nunberg et al. (1984) Proc. Natl. Acad. Sci. USA 81:3675-3679. The oligopeptide may then be screened for eliciting Ab cross-reactive with the naturally-occuring MOMP.

A number of techniques are available for synthesizing short, single-stranded DNA fragments, e.g., the phosphoramidite method described by Beaucage and Carruthers (1981) Tetrahedron Lett. 22:1859-1862. A particularly useful adaptation of the method of Beaucage and Carruthers is reported by Warner et al. (1984) DNA 3:401-411. Using the method of Urdea et al., single-stranded DNA fragments having a length of up to 100 bases may be synthesized, and double-stranded DNA fragments may be formed by annealing and ligating a plurality of single-stranded fragments under appropriate conditions. Alternatively, the complementary strand may be added using DNA polymerase with an appropriate primer sequence.

When preparing synthetic MOMP polynucleotides, it may sometimes be desirable to modify the natural nucleotide sequence. For example, it will often be preferred to use codons which are preferentially recognized by the desired host. When employing a yeast host, codons which appear at high frequency in the structural genes encoding the yeast glycolytic enzymes may be employed. In some instances, it may be desirable to further alter the nucleotide sequence to create or remove restriction sites to increase stability or to substitute one or more amino acids in the resulting polypeptide. Such changes may be made to enhance the immunogenicity of the polypeptide, facilitate conjugating the polypeptide to a carrier protein, or the like. It may also be desirable to add amino acids to the N-terminus or C-terminus of the polypeptide, where such additional amino acids provide for a desired result.

To produce a desired MOMP polypeptide, the MOMP polynucleotides will be incorporated into DNA constructs capable of being introduced into a desired expression host, usually either a prokaryotic host or eukaryotic host, such as yeast. Such DNA constructs will include the MOMP polynucleotide encoding the polypeptide product, transcriptional and translational initiation regulatory sequences joined to the 5'-end of the polynucleotide, and transcriptional and translational termination regulatory sequences joined to the 3'-end of the polynucleotide. The DNA constructs will usually also include a replication system recognized by the expression host to allow for self-replication, and will often include other functional sequences such as markers allowing for selection of transformed hosts, additional replication systems, secretory leader and processing signal sequences, and the like. The replication system, however, is not necessary since the DNA construct may allow for integration into the host genome.
Integration is facilitated by providing short DNA fragments on either side of the MOMP polynucleotide, which fragments are homologous to a desired location in the host genome.

The transcriptional initiation regulatory sequences will include a promoter region recognized by the expression host. For E. coli hosts, the lac promoter, lambda P_{L} or P_{R}, or the β-galactosidase promoter, as exemplified in the Experimental section hereinafter, are suitable. For yeast hosts, suitable promoters include those involved with the enzymes in a yeast glycolytic pathway, such as the promoters for alcohol dehydrogenase, glyceraldehyde-3-phosphate dehydrogenase, pyruvate kinase, triose phosphate isomerase, phosphoglucoisomerase, phosphofructokinase, and the like. By employing these promoters with other regulatory sequences, such as enhancers, operators, and the like, and using a host having an intact regulatory system, one can regulate the expression of the MOMP polypeptide by a number of techniques, such as varying the carbon source, e.g., replacing glucose with galactose; varying the concentration of a nutrient, e.g., acid phosphatase, or changing the temperature with a temperature sensitive promoter or regulatory system.

The transcriptional termination regulatory sequence will include a terminator, preferably a terminator balanced with the promoter to provide proper transcription. Conveniently, the terminator which is naturally found with the promoter may be employed. In the exemplary embodiment described in the Experimental section hereinafter, the MOMP polynucleotide is inserted between the β-galactosidase promoter and terminator within the β-galactosidase structural gene so that a fusion product is formed.

Enhanced yields of the polypeptides of the present invention may be obtained by employing DNA constructs which include a secretory leader and processing signal sequence to effect secretion of the gene product in yeast. The use of such secretory leader and processing signal sequences will be particularly effective with polypeptides below about 40 kilodaltons, more usually below about 30 kilodaltons, although it is expected that the system will function with polypeptides equal to the length of the whole MOMP, i.e., ranging from 38 to 45 kilodaltons. The secretory leader and processing signal sequences will normally be derived from naturally-occurring DNA sequences in yeast which provide for secretion of a polypeptide. Such polypeptides which are naturally secreted by yeast include α-factor, a-factor, acid phosphatase, and the like. If desired, the naturally-occurring sequence may be modified, for example, by reducing the number of lys-arg pairs in α-factor which define the processing site (while retaining at least one pair), or by reducing the length of the secretory leader sequence (while retaining sufficient length to provide for secretion) or by introducing point mutations, deletions or other modifications which facilitate manipulation, e.g., introducing restriction recognition sites.
Conveniently, the secretory leader and processing signal sequence may be joined to the MOMP polynucleotide by providing appropriate cohesive ends on the polynucleotide fragment, by use of appropriate adaptor molecules, or a combination of both. A portion of the structural gene for the secretory protein may be left in the final DNA construct when it is desired to produce a fused translation product, as discussed above.

Polypeptides of the present invention may also be recovered intracellularly as follows. After the transformed cell culture has reached a high density, the cells will be separated, typically by centrifugation, lysed, and the MOMP polypeptides isolated and purified by various techniques, such as extraction, affinity chromatography, electrophoresis, dialysis, and combinations thereof.

The MOMP polypeptides may also be prepared by conventional solid-phase synthesis techniques, such as those described by Merrifield (1963) J. Am. Chem. Soc. 85:2149-2156. Such solid-phase techniques are suitable for preparation of polypeptide fragments of up to about 50 to 100 amino acids, or more. Generally, however, as the length of the polypeptide increases above 25 amino acids, the difficulty in the synthesis increases and the desirability of employing a solid-phase synthesis technique diminishes.

The polypeptides of the present invention, and fragments thereof, may be employed in a variety of ways. The polypeptides can be employed both as labelled and unlabelled reagents in various immunoassays, bioassays, and the like, for the detection of Chlamydia trachomatis or antibodies to Chlamydia trachomatis in a biological sample, e.g., serum. Suitable labels include radionuclides, enzymes, fluorescers, chemiluminescers, enzyme substrates or co-factors, enzyme inhibitors, particles, dyes, and the like. Such labelled reagents may be used in a variety of well known assays, such as radioimmunoassays, enzyme immunoassays, e.g., ELISA, fluorescent immunoassays, and the like. See, for example, U.S. Patent Nos. 3,766,162; 3,791,932; 3,817,837; 3,996,345; and 4,233,402. Polypeptides of the present invention may also find use in vaccines against infection by Chlamydia trachomatis. Larger polypeptides, having a molecular weight exceeding about 5,000 daltons, may be used without further modification. Smaller haptens (i.e., those below about 5 kD), however, should be conjugated to an appropriate immunogenic carrier in order to elicit the desired immune response. Suitable immunogenic carriers include tetanus toxoid and hepatitis B surface antigen. It will be possible to link short DNA fragments expressing the MOMP polypeptides to genes expressing proteins from other pathogenic organisms or viruses. In this way, the resulting fused proteins may provide immunity against more than one disease.

In preparing a vaccine, the polypeptides will normally be incorporated in a physiologically acceptable medium, such as water, physiological saline, phosphate buffered saline, and the like. The vaccine may be administered intravenously, intraarterially, subcutaneously, intraperitoneally, or the like. The amount of immunogen employed per dose will be about 5 to 10 micrograms, if liquid, in a volume of about 0.25 to 1 ml, and may be administered repeatedly at about 2 to 4 week intervals, usually not more than 2 or 3 times.

The polynucleotides of the present invention may be employed as labelled polynucleotide probes suitable for screening biological samples for the presence of various strains of Chlamydia trachomatis. Probes comprising DNA or RNA from conserved regions of the MOMP gene may be employed for detecting a broad range of Chlamydia, while probes comprising regions of the MOMP gene specific for a particular strain may be employed to identify that strain. The polynucleotide sequences in such probes will typically be at least 12 nucleotides, more typically 16 or more nucleotides. Conveniently, the nucleotide fragment may be synthesized based on the sequence set forth in Appendix A, hereinafter.

Suitable labels include radionuclides, heavy metals, organic ligands, and the like, which allow for detection in conventional assays. Biological samples will be prepared in a conventional manner, e.g., by lysing the Chlamydia to release the nucleic acids.

### EXPERIMENTAL

The following experiments are offered by way of illustration, not by way of limitation.

### MATERIALS AND METHODS

### Reagents

DNase, RNase, endonuclease restriction enzymes, T4 ligase, kinase, DNA Polymerase I, and EcoRI methylase were obtained from Bethesda Research Laboratories. Nitrocellulose was obtained from Schleicher and Schuell. Peroxidase conjugated anti-mouse, anti-rabbit, and peroxidase anti-peroxidase (PAP) sera were obtained from Cappell. Proteinase K, isopropylthiogalactoside (IPTG), and 4-chloro-1-naphthol were from Sigma Chemical Co. Phage packaging mix was obtained from Amersham. CNBr-activated Sepharose® -4B was obtained from Pharmacia.

### Bacterial Strains

E. coli Y1088, Y1089, Y1090, and BNN 97 were obtained from R. Young and R. Davis (Stanford University). For C. trachomatis, two trachoma strains, B/TW-5/OT, and C/TW-3/OT, and one LGV strain, L₂/434/Bu, were grown in HeLa 229 cells and Renografin purified as described by Kuo et al. (1977) in: "Nongonococcal Urethritis and Related Infections," Hobson and Holmes, eds., Am. Soc. Microbiol. pp. 176-185.

### Antibodies

Polyvalent antiserum to C. trachomatis was obtained from rabbits immunized with purified LVG (L₂ serovar) organisms that were grown in chick embryo yolk sacs. Anti-E. coli reactivities in this antiserum were removed by passage through a Sepharose®-4B column derivitized with an E. coli lysate. For this purpose, approximately 20mg of DNase and RNase treated lysate of induced BNN 97 were coupled to 1mg of CNBr-activated Sepharose®-4B according to the manufacturer's instructions. The development, specificities, and ascites production of monoclonal antibodies specific for C. trachomatis have been previously reported (Stephens et al. (1982) J. Immunol. 128:1083).

### Insertion of Chlamydial DNA into λgt11

Chlamydial DNA was isolated from cell extracts of serovars L₂, B, and C by proteinase K treatment (65µg/ml, 45°C, 1 hr.) and solubilization in 1% sodium dodecyl sulfate (SDS). Following phenol extraction, the preparations were treated with 50µg/ml RNase (60°C, 30 min.), phenol/chloroform extracted, and ethanol precipitated. Standard procedures were used for enzymatic reactions and for isolation of λ phage DNA (Molecular Cloning Maniatis et al. Cold Springs Harbor Lab., 1982). Chlamydial DNA from serovar L₂ (150µg) was partially digested with DNase I as previously described (Ruther et al. (1982) Proc. Natl. Acad. Sci. USA 79:6852). Digested DNA was fractionated in a 1.25% agarose gel, and 500-2000 base pair fractions were collected on Whatman DE-81 paper and eluted as previously described (Dretzen et al. (1981) Annals of Biochem. 112:295). After treatment with DNA polymerase I, the DNA was methylated with EcoRI methylase, and 2µg of this preparation were ligated to phosphorylated EcoRI linkers with T4 ligase. These fragments were then cleaved with EcoRI endonuclease and fractionated on a Sepharose® G-150 column. Chlamydial DNA fractions were pooled and ethanol precipitated, and 20ng of the chlamydial DNA were ligated to 1µg of EcoRI cleaved λgtll. The EcoRI site is located within the β-galactosidase gene under the regulatory control of the β-galactosidase promoter and terminator. The ligated DNA was packaged into phage according to the manufacturer's instructions. Phage were plated and amplified in E. coli Y1088, and approximately 2 x 10⁵ recombinant phages were obtained.

### Screening of Recombinant Phages

E. coli Y1090 was infected with recombinant phage preparations that resulted in approximately 10⁴ plaque forming units (PFU) per 150mm plate. Plates were initially incubated at 42°C until small plaques became visible (approx. 5 hrs.). Plates were then overlayed with IPTG saturated nitrocellulose disks and incubated an additional 2 hrs. at 37°C. The nitrocellulose disks were carefully removed from the plates, rinsed in phosphate buffered saline (PBS) (pH 7.4) to remove any residual agar, and blocked in PBS containing 5% bovine serum albumin (BSA) for 60 min. at 37°C to prevent subsequent nonspecific adsorption of protein. The disks were incubated with monoclonal antibodies (1:1000 dilution in PBS containing 0.05% Tween®-20) for 2 hr., at room temperature or overnight at 4°C. The disks were washed for 1 hr. with 6 changes of PBS-Tween® and incubated with peroxidase-conjugated, anti-mouse antibody (1:2000) for 1 hr. at room temperature, followed by a 1 hr. incubation with peroxidase anti-peroxidase (PAP) (1:2000 dilution). The disks were then washed with 6 changes of PBS-Tween® followed by 2 changes of PBS. The immune reactions were detected by adding 0.5mg/ml of 4-chloro-1-naphthol and 0.001% H₂O₂ in PBS and agitated for 5-15 min. Plaques showing positive reactions were selected, plated at low densities, and reassayed with antibody. This process was repeated until all plaques were reactive.

### Analysis of Proteins by SDS-PAGE and Immunoblotting

Lysogens were produced from selected λgt11 recombinants by infecting E. coli Y1089 as previously described by Young and Davis (1983) Proc. Natl. Acad. Sci. USA 80:1194. Lysates from induced recombinant lysogens were prepared, and 20µg aliquots were electrophoresed on 7.5% or 10% SDS-polyacrylamide gels (SDS-PAGE) according to Laemmli (1970) Nature 227:680. The proteins in some gels were stained with Coomassie brilliant blue, while those from other gels were electrophoretically transferred to nitrocellulose for immunoblotting, as described by Towbin (1979) Proc. Natl. Acac. Sci. USA 76:4350-4354. Following electrophoretic transfer, nitrocellulose sheets were blocked in 5% BSA and probed with either a 1:1000 dilution of rabbit polyvalent anti-C. trachomatis antiserum or mouse ascites containing high titered monoclonal antibody. Immune reactions were detected as described above for the screening of recombinant plaques, except that the PAP step was omitted. Prestained molecular weight standards were: myosin (200,000), phosphorylase B (92,500), BSA (68,000), ovalbumin (43,000), chymotrypsinogen (25,700), lactoglobulin (18,400), and cytochrome C (12,300), (Bethesda Research Laboratories).

### Characterization of λgt11/L2/33 Insert DNA

λgt11/L2/33 insert DNA was obtained from EcoRI digests of the recombinant phage and separated on agarose gels. For dot blot hybridization, ³²P-labelled insert DNA was reacted with lysates of C. trachomatis serovars A/G-17/OT, B/TW-5/OT, Ba/AP-2/OT, C/TW-3/OT, D/UW-3/Cx, E/UW-5/Cx, F/UW-6/Cx, G/UW-57, H/UW-43/Cx, I/UW-12/Ur, J/UW-36/Cx, K/UW-53/Cx, L₁/440/Bu, L₂/434/Bu, L₃/404/Bu, C. psittaci strain Mn, and HeLa 229 host cells. Lysates were prepared from approximately 10µg of each chlamydial strain by proteinase K digestion (1mg/ml in 10mM Tris, pH 8.5, and 1mm EDTA) for 1.5 hr. at 37°C. Samples were made to 0.2N NaOH, heated to 100°C for 5 min., and placed on ice. The NaOH was neutralized with one volume of cold 0.2M acetic acid, followed by 0.5 volume of cold 20XSSC. The samples were filtered through nitrocellulose sheets and the sheets were washed with 6XSSC, air dried and baked 3 hr. at 80°C. The sheets were probed with ³²P-labelled λgt11/L2/33 insert at 65°C by standard procedures (Molecular Cloning supra.) Southern blots of BamHI-digested C. trachomatis DNA and endonuclease restriction mapping of the λgt11/L2/33 insert were performed by standard procedures (Molecular Cloning supra.).

### Insertion of Chlamydial DNA into λ 1059

A library of chlamydial genomic DNA was produced in the bacteriophage lambda 1059 system, which cloning system was described by Karn et al. (1980) Proc. Natl. Acad. Sci. USA 77:5172-5176. C. trachomatis L₂ DNA was randomized by partial digestion with endonuclease restriction enzyme Sau3A or cleaved with BamHI and ligated to BamHI digested vector. Ligated DNA was packaged in vitro, as described by Sternberg et al. (1977) Gene 1:255-280, and plated in E. coli Q359 for screening as described in Karn et al. (1980) supra. Phages were plated in E. coli Q359 at densities of approximately 3 x 10³ plaque forming units per 150mm plate. The plates were overlayed with nitrocellulose disks and the disks containing plaque adsorbed DNA were air dried and baked 3hr at 80°C. The disks were probed with ³²P labelled λgt11/L2/33 insert DNA at 60°C by standard procedures (Mololecular Cloning, supra). Several plaques that produced strong signals were picked and reassayed as above until all plaques from a clone were uniformly reactive. DNA was isolated from the selected phage recombinants by standard procedures (Molecular Cloning, supra). Two clones were mapped by endonuclease restriction analysis and Southern blotting by standard procedures (Molecular Cloning, supra). Both λ1059 recombinants had more than one BamHI insert, however, bands with identical gel mobilities were identified which were not shared with bands from the vector, and some of these bands hybridized to the insert DNA probes in Southern blots. This process permitted mapping of contiguous endonuclease restriction sites that flanked the location of the homolog to the λg t11/L2/33 insert. The may obtained by endonuclease restriction analyses was verified by generating subclones of specific fragments in a plasmid vector (pUC 18), and predicted cross-hybridizations between these clones and with the λ1059 recombinants were observed in Southern blots. Fragments that included the putative coding region and flanking regions were used for DNA sequencing.

### RESULTS

### Detection of Chlamydial Antiqens

DNA obtained from C. trachomatis serovar L₂ was partially digested with DNase I and inserted into the bacteriophage vector λgt11. The resulting plaques were transferred to nitrocellulose for the direct detection of C. trachomatis-specific antigens.
Polyvalent anti-L₂ rabbit serum detected seven plaques that produced strong immune reactions from among the 2 x 10⁴ recombinant plaques assayed. The positive plaques were replated at low densities and screened with polyvalent antiserum.

After plaque purification, the seven recombinants were tested with a pool of monoclonal antibodies. The monoclonal antibody pool consisted of four antibodies (2C1, 2G1, 2H2, AE11) that each bind a mutually exclusive MOMP determinant (Stephens et al. (1982) supra.) One of the clones, designated λgt11/L2/33, reacted with the pool of antibodies, while the other six recombinant clones did not.
Subsequently, λgt11/L2/33 was tested with each of over 15 monoclonal antibodies representing species-, subspecies-, and type-specific anti-chlamydial reaction patterns. The specificities of the antibodies and their reaction pattern with λgt/11/L2/33 are presented in Table 1. The reaction pattern demonstrated that λgt11/L2/33 was producing a polypeptide that displays species-, subspecies-, and type-specific epitopes of the chlamydial MOMP. The lack of reaction of λgt11/L2/33 to antibodies not reactive with the L₂ serovar was expected since the recombinant was derived from serovar L₂ DNA. Two antibodies (AE11 and 3H10) that do react with native L₂ MOMP did not react with the polypeptide expressed by λgt11/L2/33 using this plaque assay. The two antibodies, however, gave positive reactions with λgt11/L2/33 expressed polypeptide in immunoblotting.

**Table 1**

| Monoclonal Antibody No. | Serovar Specificities | Reaction with λgt11/L2/33 |
|---|---|---|
| 2C1, IH8 | all serovars | + |
| AE11 | all serovars except C | -* |
| 3H10 | A,B,D,E,F,G,H,K,L1,L2,L3 | -* |
| KG5 | B,D,E,F,G,H,K,L1,L2,L3 | + |
| DA10 | B,D,E,G,F,L1,L2,L3 | + |
| 2G3 | B,D,E,K,L1,L2,L3 | + |
| 2G1 | B,F,G,H,K,L2,L3 | + |
| 3H1, 2IIE3 | B,D,E,L1,L2 | + |
| JC8 | B,D,G,F,L2 | + |
| FE10 | E,G,F,L2 | + |
| JG1 | B,D,E,L2 | + |
| 2H2, 2H5 | L2 | + |
| 1B7, DD1 | B | - |
| 2B1 | C,J | - |
| FC2 | F | - |
| JG9 | D | - |

| | | |
|---|---|---|
| * Positive reaction obtained by immunoblotting. | | |

### Analysis of Recombinant Fusion Polypeptides

E. coli lysogens were prepared for each of the positive λgt11 clones to provide a source of fusion polypeptides for analysis. Lysates obtained from induced lysogens were assessed by Coomassie blue stained PAGE gels and by immunoblotting of the proteins that were electrophoretically transferred from PAGE gels to nitrocellulose. The molecular weights of these fusion proteins were estimated to range from 132,000 to 146,000.

Immunoblot analysis of PAGE gels using polyvalent rabbit anti-L₂ revealed that each of the seven clones produced strong reactions in the plaque assay. The λgt11/L2/33 product stained most intensely, while the products from two other recombinants stained very faintly. Immunoblot analysis was also performed with the monoclonal antibodies. Of the seven recombinants, only λgt11/L2/33 reacted with monoclonal antibodies as expected from the results on the plaque assays with these same antibodies. The monoclonal antibodies that recognized species-specific and subspecies-specific determinants on L₂ chlamydial MOMP reacted strongly with the polypeptide produced by λgt11/L2/33, while the L₂ type-specific monoclonal antibodies produced negative or equivocal reactions.

### Characterization of λgt11/L2/33 Insert DNA

DNA from the λgt11/L2/33 recombinant was isolated, labelled with ³²P, and used to probe dot blots of each of the 15 C. trachomatis serovars, the Mn strain of C. psittaci, and HeLa 229 host cells. Reactions were detected with all chlamydiae but not with HeLa 229 host cell DNA. Furthermore, Southern blots of BamHI digests of C. trachomatis DNA obtained from serovars L₂, B, and C revealed one fragment in each preparation which reacted with ³²P-labelled insert DNA from λgt11/L2/33. The molecular weight of this fragment varied slightly between serovars but was approximately 9.4 kb.

Preparations of λgt11/L2/33 insert DNA were obtained from EcoRI digests and separated on agarose gels. The insert was estimated to be about 1.1kb in length with restriction sites for HaeII, HaeIII, HhaI, and XhoI. Restriction sites for AccI, BamHI, BclI, BstEII, EcoRI, EcoRV, PstI, PvuI, SstI, and SstII were not detected.

The approximately 1.1 kb insert DNA was sequenced by standard techniques, and the sequence is set forth in Appendix A.

### Sequencing of λ 1059 Inserts

Lambda 1059 recombinants having 9.2 to 9.8 kb inserts that were shown to be homologous with λgt11/L2/33 by Southern analysis were used for endonuclease restriction mapping, and additional Southern analyses. Two contiguous fragments (BamHI/EcoRI and EcoRI/EcoRI) were identified, and these contain sufficient base pairs to encode for the L₂ MOMP gene product. These fragments were cloned into M13 for DNA sequencing. The sequence data for a 9.2 kb fragment (designated L2 B9-F DNA) are set forth in Appendix B.

The sequence includes an untranslated region comprising 1287 bases, followed by a 66 base region encoding a 22 amino acid leader sequence. Coding for the MOMP begins at base 67 (amino acid 23) and extends through base number 1182 (amino acid 394). The molecular weight for the MOMP including the leader is calculated to be 42,557 daltons.

The N-terminus of the MOMP was located on the basis of the 25 amino acid N-terminus reported by Nano et al. (1985) supra. Differences in the sequences of the N-terminus reported by Nano et al. and that reported herein are found at amino acid residues 32, 44, and 45, as numbered in Appendix B. These differences may result from differences among the isolates or mistakes in amino acid sequencing.

The sequence set forth in Appendix A corresponds to amino acids 247 through the 3'-terminus in Appendix B, with certain deviations. Bases 36-38 in Appendix A are AGA, corresponding to amino acids GlyGlu, while bases 773-775 in Appendix B are TGT, corresponding to amino acids GlyVal. These deviations are underlined in both Appendices. The DNA sequence corresponding to amino acids 305 through 394 in Appendix B has several deviations from Appendix A which result in a different reading frame for the sequence of Appendix B. Base numbers 174, 181, and 186 in Appendix A were not detected in the λ 1059 clones. Base number 35 in Appendix A is a T, while the corresponding base in Appendix B (in amino acid 357) is a C. Finally, a G is inserted in amino acid 358 and a G is inserted in amino acid 374 in the sequence of Appendix B. In both Appendices A and B, bases which are inserted or changed relative to the other Appendix are boxed, while deleted bases are indicated by an arrow. Both the DNA and amino acid sequences of Appendix B are believed to be correct.

According to the subject invention, novel recombinant DNA constructs are provided for the expression of a polypeptide having immunological activity corresponding to that of a naturally-occurring major outer membrane protein of Chlamydia trachomatis. Such polypeptides may find use as reagents in the detection of Chlamydia trachomatis or antibodies to Chlamydia trachomatis, and as vaccines against infection by Chlamydia trachomatis in susceptible hosts.

GluPheProLeuAspLeuLysAlaGlyThrAspGlyGluThrGlyThrLysAspAlaSer
GAATTCCCTCTTGATCTTAAAGCAGGAACAGATGGAGAGACAGGAACTAAGGATGCCTCT
CTTAAGGGAGAACTAGAATTTCGTCCTTGTCTACCTCTCTGTCCTTGATTCCTACGGAGA
^     ^     ^                                  ^   ^    ^
1 ecor1, 7 mnl1, 13 mbo1 sau3a, 47 dde1, 51 fok1, 56 mnl1,

IleAspTyrHisGluTrpGlnAlaSerLeuAlaLeuSerTyrArgLeuAsnMetPheThr
ATTGATTACCATGAATGGCAAGCAAGTTTAGCTCTCTCTTACAGACTGAATATGTTCACT
TAACTAATGGTACTTACCGTTCGTTCAAATCGAGAGAGAATGTCTGACTTATACAAGTGA
                              ^                 ^
90 alu1, 108 xmn1,

ProTyrIleGlyValLysTrpSerArgAlaSerPheAspAlaAspThrIleArgTyrCys
CCCTACATTGGAGTTAAATGGTCTCGAGCAAGTTTTGATGCAGACACGATTCCGTATTGC
GGGATGTAACCTCAATTTACCAGAGCTCGTTCAAAACTACGTCTGTGCTAAGGCATAACG
                        ^^                      ^           ^
143 ava1 xho1, 144 taq1, 168 hinf1, 180 mnl1,

LeuSerProLysSerAlaThrThrValPheAspValThrThrLeuAsnProThrIleAla
GTCAGCCCGAAGTCAGCTACAACTGTCTTTGATGTTACCACTCTGAACCCAACTATTGCT
CAGTCGGGCTTCAGTCGATGTTGACAGAAACTACAATGGTGAGACTTGGGTTGATAACGA
^              ^
181 dde1, 195 alu1,

GlyAlaGlyAspValLysAlaSerAlaGluGlyGlnLeuGlyAspThrMetGlnIleVal
GGAGCTGGCGATGTGAAAGCTAGCGCAGAGGGTCAGCTCGGAGATACCATGCAAATCGTT
CCTCGACCGCTACACTTTCGATCGCGTCTCCCAGTCGAGCCTCTATGGTACGTTTAGCAA
  ^              ^    ^    ^     ^
243 alu1, 258 alu1, 263 hha1, 268 mnl1, 275 alu1,

SerLeuGlnLeuAsnLysMetLysSerArgLysPheSerValLeuGlnAM
TCCTTGCAATTGAACAAGATGAAATCTAGAAAATTTTCGGTATTGCAGTAGGAACAACTA
AGGAACGTTAACTTGTTCTACTTTAGATCTTTTAAAAGCCATAACGTCATCCTTGTTGAT
                         ^    ^
325 xba1, 329 xmn1,

TTGTGGATGCAGACAAATACGCATTACAGTTGAGACTCGCTTGATCGATGAGAGAGCTGC
AACACCTACGTCTGTTTATGCGTAATGTCAACTCTGAGCGAACTAGCTACTCTCTCGACG
    ^                            ^        ^^^         ^^
365 fok1, 394 hinf1, 403 mbo1 sau3a, 404 cla1, 405 taq1, 415
 alu1, 416 bbv fnu4h1,

TCACGTAAATGCACAATTCCGCTTCTAATTAATTGTATAATTTTGTTAAACTTTGGCAAG
AGTGCATTTACGTGTTAAGGCGAAGATTAATTAACATATTAAAACAATTTGAAACCGTTC

TTTATCTTTGTTAATAACGTTAATAACACTATCCGTGTTTCTGGGCTCGACTTCGGTCGG
AAATAGAAACAATTATTGCAATTATTGTGATAGGCACAAAGACCCGAGCTGAAGCCAGCC
                                          ^    ^          ^^
523 ban2 hgiJ11 sduI, 527 taq1, 539 ban1, 540 asu1 ava2,

GTCCAGTTTTTTTTGCAAAAATTTTTTTTCTTACTTTCGATCTCCCTCCTATCTCTCTTA
CAGGTCAAAAAAAACGTTTTTAAAAAAAAGAATGAAAGCTAGAGGGAGGATAGAGAGAAT
                                     ^ ^    ^
577 taq1, 579 mbo1 sau3a, 585 mnl1,

CAACAAAATCTAAAATTTCTCTAAAAGAAGATTGCATAAAAGGCCTCTTTCCAGTACTAT
GTTGTTTTAGATTTTAAAGAGATTTTCTTCTAACGTATTTTCCGGAGAAAGGTCATGATA
                          ^              ^^ ^         ^^
627 mbo11, 641 hae1 stu1, 642 hae111, 644 mnl1, 653 sca1, 65
4 rsa1,

ATCGGTCTACTTGAGCGCGCCCGTAGCTCAATGGTAGAGCTGTAGCCTTCCAAGCTACCG
TAGCCAGATGAACTCGCGCGGGCATCGAGTTACCATCTCGACATCGGAAGGTTCGATGGC
    ^         ^^^       ^           ^              ^    ^
665 acc1, 675 hha1, 676 tac1, 677 hha1, 685 alu1, 698 alu1,
713 alu1, 718 hpa11,

APPENDIX B

GGATCCTCACCCTCTTCATAAGCACGAATGCATTCTCTTAGGTTTCCTAACTCCCTCGT
AATTTTTCTATGTTCTTCTGCGCTAATAGGTCCGACATACCCCAACAAATCAGCTACTG
TGCGCCCTTCAGGATAATGTCTGCGAACCGAAGATTCGACATGCAATCCTGGCCAATCC
TTCTCCAGCATCTTGAGCCTTAAAAAGTACGCTCAGATACATTAGGTCTGAAGGATGT
AAGGTACAGACCCTAATACAGAAGCTTTGGCATGGATCGTATCTTCAACGAAATCACGG
TCCATATGCAGCTCTTGAGCCAAAAAATCTTGCAAATTTTTTAATGTAATCCTTACGAA
CAGGAACAAGCCTCTTATTCCCTTGCTCATCTGTATGCCAAACACGCGTCGGAATATCA
CGTATCGCTCGATAAGAGATCCCCACATTATATTGTAAAACGTTTTCTGCCAGCGTTTT
ACCAAAACGATCACATACTCCTGCGCGATCACAATGCTCAGGAACACTTCTACGCTGAG
GACGATAAGCTTCTTCCTTCTTTTTCTCATGCTGCACAACAGCAACATGCCATATACGC
AAAGTGATGATAGACAAAGCAATAATAAATCCCACAAGTAGTCTGTTAGCCTTTTCTGG
CACAGAAAGTGGGGTGCGTCGTTTTCTTTTCATATATGGTTAGTTTAATCTGTTTTTAT
TGGTCGACCGTTTAAAAACACTTTCTTTGTAGTAATAAAAACGATTTCTATCAAAACAA
ATTCTTAGATTTTCTTACAAAAATCTCCTCTTTTCTTTTAGCCAAACCCCCATCTTCGA
GCTATTCCAAACACAAAAATCTTAGGTTTTGGAAATTAACAACTCATAAAAATTGAACT
GTTTTGTAATTAACTCAAAACCCTCTCATTCTCAACAATCAACATATTGCCAATATGGC
TTTTGCTCTCGGTTTCAGAGCGATTTTTTTCGCAAAAACCAAGAACATAAAACATAAAA
AGATATACAAAAATGGCTCTCTGCTTTATCGCTAAATCAGGAGGCGCTTAAGGGCTTCT
TCCTGGGACCGAACGTTTTTCTTATCTTCTTTACGACAATAAGAAAATTTTGTTATGGCT
CGAGCATTGAACGACATGTTCTCGATTAAGGCTGCTTTTACTTGCAAGACATTCCTCAG
GCCATTAATTGCTACAGGACATCTTGTCTGGCTTTAACTAGGACGCAGTGCCGCCAGAA

                                                                                    1
                                                                                    Met Lys
AAACATAGCGAGCACAAACAGAGCTAATTATACAATTTAGAGCTAAGA     ATG AAA

                                        10
Lys Leu Leu Lys Ser Val Leu Val Phe Ala Ala Leu Ser Ser Ala
AAA CTC TTG AAA TCG GTA TTA GTG TTT GCC GCT TTG AGT TCT GCT

          20                                                  30
Ser Ser Leu Gln Ala Leu Pro Val Gly Asn Pro Ala Glu Pro Ser
TCC TCC TTG CAA GCT CTG CCT GTG GGG AAT CCT GCT GAA CCA AGC

                                        40
Leu Met Ile Asp Gly Ile Leu Trp Glu Gly Phe Gly Gly Asp Pro
CTT ATG ATC GAC GGA ATT CTA TGG GAA GGT TTC GGC GGA GAT CCT

          50                                                  60
Cys Asp Pro Cys Thr Thr Trp Cys Asp Ala Ile Ser Met Arg Met
TGC GAT CCT TGC ACC ACT TGG TGT GAC GCT ATC AGC ATG CGT ATG

                                        70
Gly Tyr Tyr Gly Asp Phe Val Phe Asp Arg Val Leu Gln Thr Asp
GGT TAC TAT GGT GAC TTT GTT TTC GAC CGT GTT TTG CAA ACA GAT

          80                                                  90
Val Asn Lys Glu Phe Gln Met Gly Ala Lys Pro Thr Thr Ala Thr
GTG AAT AAA GAA TTC CAA ATG GGT GCC AAG CCT ACA ACT GCT ACA

100
Gly Asn Ala Ala Ala Pro Ser Thr Cys Thr Ala Arg Glu Asn Pro
GGC AAT GCT GCA GCT CCA TCC ACT TGT ACA GCA AGA GAG AAT CCT

110 120
Ala Tyr Gly Arg His Met Gln Asp Ala Glu Met Phe Thr Asn Ala
GCT TAC GGC CGA CAT ATG CAG GAT GCT GAG ATG TTT ACA AAT GCT

130
Ala Tyr Met Ala Leu Asn Ile Trp Asp Arg Phe Asp Val Phe Cys
GCT TAC ATG GCA TTG AAT ATT TGG GAT CGT TTT GAT GTA TTC TGT

140 150
Thr Leu Gly Ala Thr Ser Gly Tyr Leu Lys Gly Asn Ser Ala Ser
ACA TTA GGA GCC ACC AGT GGA TAT CTT AAA GGA AAT TCA GCA TCT

160
Phe Asn Leu Val Gly Leu Phe Gly Asp Asn Glu Asn His Ala Thr
TTC AAC TTA GTT GGC TTA TTC GGA GAT AAT GAG AAC CAT GCT ACA

170 180
Val Ser Asp Ser Lys Leu Val Pro Asn Met Ser Leu Asp Gln Ser
GTT TCA GAT AGT AAG CTT GTA CCA AAT ATG AGC TTA GAT CAA TCT

190
Val Val Glu Leu Tyr Thr Asp Thr Thr Phe Ala Trp Ser Ala Gly
GTT GTT GAG TTG TAT ACA GAT ACT ACT TTT GCT TGG AGT GCT GGA

200 210
Ala Arg Ala Ala Leu Trp Glu Cys Gly Cys Ala Thr Leu Gly Ala
GCT CGT GCA GCT TTG TGG GAA TGT GGA TGC GCG ACT TTA GGC GCT

220
Ser Phe Gln Tyr Ala Gln Ser Lys Pro Lys Val Glu Glu Leu Asn
TCT TTC CAA TAC GCT CAA TCC AAG CCT AAA GTC GAA GAA TTA AAC

230 240
Val Leu Cys Asn Ala Ala Glu Phe Thr Ile Asn Lys Pro Lys Gly
GTT CTC TGT AAC GCA GCT GAG TTT ACT ATC AAT AAG CCT AAA GGA

250
Tyr Val Gly Gln Glu Phe Pro Leu Asp Leu Lys Ala Gly Thr Asp
TAT GTA GGG CAA GAA TTC CCT CTT GAT CTT AAA GCA GGA ACA GAT

260 270
Gly Val Thr Gly Thr Lys Asp Ala Ser Ile Asp Tyr His Glu Trp
GGT GTG ACA GGA ACT AAG GAT GCC TCT ATT GAT TAC CAT CAA TGG

280
Gln Ala Ser Leu Ala Leu Ser Tyr Arg Leu Asn Met Phe Thr Pro
CAA GCA AGT TTA GCT CTC TCT TAC AGA CTG AAT ATG TTC ACT CCC

290 300
Tyr Ile Gly Val Lys Trp Ser Arg Ala Ser Phe Asp Ala Asp Thr
TAC ATT GGA GTT AAA TGG TCT CGA GCA AGT TTT GAT GCA GAC ACG

310
Ile Arg Ile Ala Gln Pro Lys Ser Ala Thr Thr Val Phe Asp Val
ATT CGT ATT GCT CAG CCG AAG TCA GCT ACA ACT GTC TTT GAT GTT

320 330
Thr Thr Leu Asn Pro Thr Ile Ala Gly Ala Gly Asp Val Lys Ala
ACC ACT CTG AAC CCA ACT ATT GCT GGA GCT GGC GAT GTG AAA GCT

340
Ser Ala Glu Gly Gln Leu Gly Asp Thr Met Gln Ile Val Ser Leu
AGC GCA GAG GGT CAG CTC GGA GAT ACC ATG CAA ATC GTT TCC TTG

350 360
Gln Leu Asn Lys Met Lys Ser Arg Lys Ser Cys Gly Ile Ala Val
CAA TTG AAC AAG ATG AAA TCT AGA AAA T[?]T T[?]C GGT ATT GCA GTA

370
Gly Thr Thr Ile Val Asp Ala Asp Lys Tyr Ala Val Thr Val Glu
GGA ACA ACT ATT GTG GAT GCA GAC AAA TAC GCA GTT ACA GTT GAG

380                                                    390
Thr Arg Leu Ile Asp Glu Arg Ala Ala His Val Asn Ala Gln Phe
ACT CGC TTG ATC GAT GAG AGA GCT GCT CAC GTA AAT GCA CAA TTC

    394
Arg Phe OC
CGC TTC TAA TTAATTGTATAATTTTGTTAAACTTTGGCAAGTTTATCTTTGTTAATA

ACGTTAATAACACTATCCGTGTTTCTGGGCTCGACTTCGGTCGGGTCCAGTTTTTTTTGC

AAAAATTTTTTTTCTTACTTTCGATCTCCCTCCTATCTCTCTTACAACAAAATCTAAAAT

TTCTCTAAAAGAAGATTGCATAAAAGGCCTCTTTCCAGTACTATATCGGTCTACTTGAC[?]

GCGCCCGTAGCTCAATGGTAGAGCTGTAGCCTTCCAAGCTACCGGTGTCAGTTCGATTCT

GATCGGGCTCTTTTTTACTCCTGTATGACTCCCAAGTCTGAAATCTGAGCGTCTCTCAGA

TGCCTTGTTAACACATAAAAAGAGGAACAAAGCTTGGAACTTTCCTGCAAACTCACTTTA

AAAGAACTATTAGAATCCGGGGCACATTTTGGACACCAGACAAGTCGCTGGAATCCCAAG

ATGAAGCCTTTTATTTTTGAAGAAAAAAATGGCCTTTACATCATCGACTTGGCTAAAACT

TTAGGTCAGTTGAAAAAGGCTGTTTCTTGCATTCAAAAAACTATCGATCAAGAGAGGTCT

ATTTTTGTTTGTTGGAACAAAAAAACAAGCAAAACAGATCATTAGAGAAGCTGCTATCGA

ATGTGGCGAATTC

## Claims (Claims for the following Contracting State(s): BE, CH, DE, FR, GB, IT, LI, LU, NL, SE)

1. A DNA construct for the expression of Chlamydia trachomatis MOMP polypeptides, which comprises the following operably linked elements:
a transcriptional promoter;
a DNA molecule encoding a C. trachomatis MOMP polypeptide comprising a MOMP polynucleotide at least 27 base pairs in length from sequence of Appendix A;
and
a transcriptional terminator, wherein at least one of said transcriptional or translational regulatory sequences is not derived from Chlamydia trachomatis.

2. The DNA construct as in Claim 1, wherein the MOMP polynucleotide encodes for substantially the entire length of the major outer membrane protein.

3. The DNA construct of claim 1, wherein the MOMP polynucleotide encodes a polypeptide reactive with antibodies specific to Chlamydia.

4. The DNA construct of claim 1, wherein the MOMP polynucleotide encodes a polypeptide reactive with antibodies specific for C. trachomatis.

5. The DNA construct of claim 1, wherein the MOMP polynucleotide encodes a polypeptide reactive with antibodies specific for a serovar of C. trachomatis.

6. A DNA construct for the expression of Chlamydia trachomatis MOMP polypeptides, which comprises the following operably linked elements:
a transcriptional promoter;
a DNA MOMP polynucleotide molecule coding for at least nine consecutive amino acids of the amino acid sequence of Chlamydia trachomatis MOMP from Appendix A, wherein the MOMP polynucleotide is fused to a second structural gene in proper reading frame therewith and is under the regulatory control of the regulatory system of the second structural gene; and
a transcriptional terminator, wherein at least one of said transcriptional regulatory sequences is not derived from Chlamydia trachomatis.

7. A DNA construct as in Claim 6, wherein the second structural gene codes for an immunogen.

8. A DNA construct as in Claim 6, wherein the second structural gene is a β-galactosidase gene.

9. An isolated polynucleotide molecule comprising at least 27 base pairs in length from the sequence of Appendix A and encoding a polypeptide having immunological cross-reactivity with the major outer membrane protein of Chlamydia trachomatis, said polynucleotide lacking at least one natural flanking region.

10. An isolated polynucleotide as in Claim 9, wherein the polypeptide encoded thereby is immunologically cross-reactive with serovar L2 MOMP.

11. An isolated polynucleotide probe for detecting the presence of Chlamydia in a biological sample, said probe comprising a polynucleotide fragment of at least 12 nucleotides from the C. trachomatis MOMP nucleotide sequence of Appendix A, which probe is capable of binding a DNA or RNA sequence characteristic of Chlamydia.

12. The isolated polynucleotide probe of claim 11, wherein the probe is capable of binding a DNA or RNA sequence characteristic of C. trachomatis.

13. The isolated polynucleotide probe of claim 11, wherein the probe is capable of binding a DNA or RNA sequence characteristic of a serovar of C. trachomatis.

14. The polynucleotide probe of claim 13, wherein said polynucleotide fragment further comprises a detectable label.

15. A cultured cell transformed or transfected by the DNA construct of claim 1 or 6.

16. A transformed cell according to claim 15, which is E. coli.

17. A peptide which comprises from nine to fifty amino acids having a sequence coming within the amino acid sequence of Appendix A and which peptide is capable of reacting with antibodies which react with the major outer membrane protein of C. trachomatis.

18. The peptide of claim 17, for use in a immunoassay for the detection of antibodies to C. trachomatis or C. trachomatis antigen in a biological sample.

19. The peptide of claim 17, which is conjugated to a carrier.

20. An immunoassay for the detection of Chlamydia trachomatis antigen or antibodies to C. trachomatis in a biological sample, said immunoassay characterized by the use of a C. trachomatis MOMP polypeptide or fragment thereof encoded by the DNA construct of claim 1 or 6, where the polypeptide may be labelled or unlabelled.

## Claims (Claims for the following Contracting State(s): AT)

1. A process for the expression of Chlamydia trachomatis MOMP polypeptides, characterized by the use of a DNA construct which comprises the following operably linked elements:
a transcriptional promoter;
a DNA molecule encoding a C. trachomatis MOMP polypeptide comprising a MOMP polynucleotide at least 27 base pairs in length from sequence of Appendix A;
and
a transcriptional terminator, wherein at least one of said transcriptional or translational regulatory sequences is not derived from Chlamydia trachomatis.

2. A process according to claim 1, wherein the MOMP polynucleotide encodes for substantially the entire length of the major outer membrane protein.

3. A process according to claim 1, wherein the MOMP polynucleotide encodes a polypeptide reactive with antibodies specific to Chlamydia.

4. A process according to claim 1, wherein the MOMP polynucleotide encodes a polypeptide reactive with antibodies specific for C. trachomatis.

5. A process according to claim 1, wherein the MOMP polynucleotide encodes a polypeptide reactive with antibodies specific for a serovar of C. trachomatis.

6. A process for the expression of Chlamydia trachomatis MOMP polypeptides, characterized by the use of a DNA construct which comprises the following operably linked elements:
a transcriptional promoter;
a DNA MOMP polynucleotide molecule coding for at least nine consecutive amino acids of the amino acid sequence of Chlamydia trachomatis MOMP from Appendix A, wherein the MOMP polynucleotide is fused to a second structural gene in proper reading frame therewith and is under the regulatory control of the regulatory system of the second structural gene; and
a transcriptional terminator, wherein at least one of said transcriptional regulatory sequences is not derived from Chlamydia trachomatis.

7. A process according to claim 6, wherein the second structural gene codes for an immunogen.

8. A process according to claim 6, wherein the second structural gene is a β-galactosidase gene.

9. A process for detecting the presence of Chlamydia trachomatis in a biological sample, characterized by the use an isolated polynucleotide probe, said probe comprising a polynucleotide fragment of at least 12 nucleotides from the Chlamydia trachomatis MOMP nucleotide sequence of Appendix A, which probe is capable of binding a DNA or RNA sequence characteristic of Chlamydia trachomatis.

10. A process according to claim 9, wherein the probe is capable of binding a DNA or RNA sequence characteristic of C. trachomatis.

11. A process according to claim 9, wherein the probe is capable of binding a DNA or RNA sequence characteristic of a serovar of C. trachomatis.

12. A process according to claim 11, wherein said polynucleotide fragment further comprises a detectable label.

13. A process for the detection of antibodies to C. trachomatis or C. trachomatis antigen in a biological sample, characterized by the use of an immunoassay comprising a peptide which comprises from nine to fifty amino acids having a sequence coming within the amino acid sequence of Appendix A and which peptide is capable of reacting with antibodies which react with the major outer membrane protein of C. trachomatis.

14. A process according to claim 13, wherein the peptide is conjugated to a carrier.

15. A process for the detection of Chlamydia trachomatis antigen or antibodies to C. trachomatis in a biological sample, characterized by the use of a C. trachomatis MOMP polypeptide or fragment thereof encoded by a DNA construct, which comprises the following operably linked elements:
a transcriptional promoter;
a DNA molecule encoding a C. trachomatis MOMP polypeptide comprising a MOMP polynucleotide at least 27 base pairs in length from sequence of Appendix A;
and
a transcriptional terminator, wherein at least one of said transcriptional or translational regulatory sequences is not derived from Chlamydia trachomatis.

16. A process for the detection of Chlamydia trachomatis antigen or antibodies to C. trachomatis in a biological sample, characterized by the use of a C. trachomatis MOMP polypeptide or fragment thereof encoded by a DNA construct, which comprises the following operably linked elements:
a transcriptional promoter;
a DNA MOMP polynucleotide molecule coding for at least nine consecutive amino acids of the amino acid sequence of Chlamydia trachomatis MOMP from Appendix A, wherein the MOMP polynucleotide is fused to a second structural gene in proper reading frame therewith and is under the regulatory control of the regulatory system of the second structural gene; and
a transcriptional terminator, wherein at least one of said transcriptional regulatory sequences is not derived from Chlamydia trachomatis.

## Patentansprüche (Patentansprüche für folgende(n) Vertragsstaat(en): BE, CH, DE, FR, GB, IT, LI, LU, NL, SE)

1. DNA-Konstrukt zur Expression von Chlamydia trachomatis MOMP-Polypeptiden, das die folgenden operabel verbundenen Elemente aufweist:
ein Transkripitionspromotor ;
ein DNA-Molekül, das ein C. trachomatis MOMP-Polypeptid kodiert und ein MOMP-Polynukleotid mit einer Länge von wenigstens 27 Basenpaaren der Sequenz aus Anhang A aufweist;
und
ein Transkriptionsterminator, wobei wenigstens eine dieser transkriptionalen oder translationalen regulativen Sequenzen sich nicht von Chlamydia trachomatis ableitet.

2. DNA-Konstrukt nach Anspruch 1, bei dem das MOMP-Polynukleotid im wesentlichen die gesamte Länge des Hauptproteins der Außenmembran kodiert.

3. DNA-Konstrukt nach Anspruch 1, bei dem das MOMP-Polynukleotid ein Polypeptid kodiert, das reaktionsfähig mit für Chlamydia spezifischen Antikörpern ist.

4. DNA-Konstrukt nach Anspruch 1, bei dem das MOMP-Polynukleotid ein Polypeptid kodiert, das reaktionsfähig mit für C. trachomatis spezifischen Antikörpern ist.

5. DNA-Konstrukt nach Anspruch 1, bei dem das MOMP-Polynukleotid ein Polypeptid kodiert, das reaktionsfähig mit für einen Serovar des C. trachomatis spezifischen Antikörpern ist.

6. DNA-Konstrukt zur Expression von Chlamydia trachomatis MOMP-Polypeptiden, das die folgenden operabel verbundenen Elemente aufweist:
ein Transkriptionspromotor ;
ein DNA-MOMP-Polynukleotid-Molekül, das wenigstens neun aufeinander folgende Aminosäuren der Aminosäuresequenz des Chlamydia trachomatis MOMP aus Anhang A kodiert, wobei das MOMP-Polynukleotid mit einem zweiten Strukturgen in dem richtigen Leseraster fusioniert ist und sich unter der regulativen Kontrolle des regulierenden Systems des zweiten Strukturgens befindet;
und
ein Transkriptionsterminator, wobei wenigstens eine dieser transkriptionalen regulativen Sequenzen sich nicht von Chlamydia trachomatis ableitet.

7. DNA-Konstrukt nach Anspruch 6, bei dem das zweite Strukturgen ein Immunogen kodiert.

8. DNA-Konstrukt nach Anspruch 6, bei dem das zweite Strukturgen ein β-Galaktosidase Gen ist.

9. Isoliertes Polynukleotidmolekül das einen Abschnitt von wenigstens 27 Basenpaaren der Sequenz aus Anhang A aufweist und ein Polypeptid kodiert, das eine immunologische Kreuz-Reaktivität mit dem Hauptprotein der Außenmembran von Chlamydia trachomatis aufweist, wobei diesem Polynukleotid wenigstens ein natürlich flankierender Bereich fehlt.

10. Isoliertes Polynukleotid wie in Anspruch 9, wobei das dadurch kodierte Polypeptid immunolgisch kreuz-reaktiv mit Serovar L2 MOMP ist.

11. Isolierte Polynukleotidsonde zum Nachweis der Anwesenheit von Chlamydia in einer biologischen Probe, wobei diese Sonde ein Polynukleotidfragment von wenigstens 12 Nukleotiden aus der C. trachomatis MOMP-Nukleotidsequenz aus Anhang A aufweist, und in der Lage ist, eine DNA- oder RNA-Sequenz, die für Chlamydia charakteristisch ist, zu binden.

12. Isolierte Polynukleotidsonde nach Anspruch 11, die in der Lage ist, eine DNA- oder RNA-Sequenz, die für C. trachomatis charakteristisch ist, zu binden.

13. Isolierte Polynukleotidsonde nach Anspruch 11, die in der Lage ist, eine DNA- oder RNA-Sequenz, die für einen Serovar des C. trachomatis charakteristisch ist, zu binden.

14. Polynukleotidsonde nach Anspruch 13, bei der das Polynukleotidfragment zusätzlich eine detektierbare Markierung aufweist.

15. Kultivierte Zelle, die durch das DNA-Konstrukt des Anspruchs 1 oder 6 transformiert oder transfiziert wurde.

16. Transformierte Zelle gemäß Anspruch 15, die E. coli ist.

17. Peptid mit neun bis fünfzig Aminosäuren, die eine Sequenz haben, die innerhalb der Aminosäuresequenz aus Anhang A vorkommt, und das ist in der Lage ist, mit Antikörpern zu reagieren, die mit dem Hauptprotein der Außenmembran der C. trachomatis reagieren.

18. Peptid nach Anspruch 17 für die Verwendung in einem Immunassay für den Nachweis von Antikörpern gegen C. trachomatis oder C. trachomatis Antigen in einer biologischen Probe.

19. Peptid nach Anspruch 17, das mit einem Träger verbunden ist.

20. Immunassay zum Nachweis von Chlamydia trachomatis Antigen oder Antikörpern gegen C. trachomatis in einer biologischen Probe, gekennzeichnet durch die Verwendung eines C. trachomatis MOMP-Polypeptides oder eines Fragments davon, das durch das DNA-Konstrukt der Ansprüche 1 oder 6 kodiert wird, wobei das Polypeptid markiert oder nicht markiert sein kann.

## Patentansprüche (Patentansprüche für folgende(n) Vertragsstaat(en): AT)

1. Verfahren zur Expression von Chlamydia trachomatis MOMP-Polypeptiden, das durch die Verwendung eines DNA-Konstrukts charakterisiert ist, das die folgenden operabel verbundenen Elemente aufweist:
ein Transkripitionspromotor ;
ein DNA-Molekül, das ein C. trachomatis MOMP-Polypeptid kodiert und ein MOMP-Polynukleotid mit einer Länge von wenigsten 27 Basenpaaren der Sequenz aus Anhang A aufweist;
und
ein Transkriptionsterminator, wobei wenigstens eine dieser transkriptionalen- oder translationalen regulativen Sequenzen sich nicht von Chlamydia trachomatis ableitet.

2. Verfahren gemäß Anspruch 1, bei dem das MOMP-Polynukleotid im wesentlichen die gesamte Länge des Hauptproteins der Außenmembran (major outer membrane protein) kodiert.

3. Verfahren gemäß Anspruch 1, bei dem das MOMP-Polynukleotid ein Polypeptid kodiert, das reaktionsfähig mit für Chlamydia spezifischen Antikörpern ist.

4. Verfahren gemäß Anspruch 1, bei dem das MOMP-Polynukleotid ein Polypeptid kodiert, das reaktionsfähig mit für C. trachomatis spezifischen Antikörpern ist.

5. Verfahren gemäß Anspruch 1, bei dem das MOMP-Polynukleotid ein Polypeptid kodiert, das reaktionsfähig mit für einen Serovar des C. trachomatis spezifischen Antikörpern ist.

6. Verfahren zur Expression von Chlamydia trachomatis MOMP-Polypeptiden, das durch die Verwendung eines DNA-Konstruktes charakterisiert ist, das die folgenden operabel verbundenen Elemente aufweist:
ein Transkripitionspromotor ;
ein DNA-MOMP-Polynukleotid-Molekül, das wenigstens neun aufeinander folgende Aminosäuren der Aminosäuresequenz des Chlamydia trachomatis MOMP aus Anhang A kodiert, wobei das MOMP-Polynukleotid mit einem zweiten Strukturgen in dem richtigen Leseraster fusioniert ist und sich unter der regulativen Kontrolle des regulierenden Systems des zweiten Strukturgens befindet;
und
ein Transkriptionsterminator, wobei wenigstens eine dieser transkriptionalen- oder translationalen regulativen Sequenzen sich nicht von Chlamydia trachomatis ableitet.

7. Verfahren gemäß Anspruch 6, bei dem das zweite Strukturgen ein Immunogen kodiert.

8. Verfahren gemäß Anspruch 6, bei dem das zweite Strukturgen ein β-Galaktosidase Gen ist.

9. Verfahren zum Nachweis der Anwesenheit von Chlamydia trachomatis in einer biologischen Probe, gekennzeichnet durch die Verwendung einer isolierten Polynukleotidsonde, wobei diese Sonde ein Polynukleotidfragment von wenigstens 12 Nukleotiden aus der Chlamydia trachomatis MOMP-Nukleotidsequenz aus Anhang A aufweist und in der Lage ist, eine DNA- oder RNA-Sequenz, die für Chlamydia trachomatis charakteristisch ist, zu binden.

10. Verfahren gemäß Anspruch 9, bei dem die Sonde in der Lage ist, eine DNA- oder RNA-Sequenz, die für C. trachomatis charakteristisch ist, zu binden.

11. Verfahren gemäß Anspruch 9, bei dem die Sonde in der Lage ist, eine DNA- oder RNA-Sequenz, die für einen Serovar des C. trachomatis charakteristisch ist, zu binden.

12. Verfahren gemäß Anspruch 11, bei dem zu diesem Polynukleotidfragment zusätzlich eine detektierbare Markierung gehört.

13. Verfahren zum Nachweis von Antikörpern gegen C. trachomatis oder C. trachomatis Antigen in einer biologischen Probe, gekennzeichnet durch die Verwendung eines Immunassays, der ein Peptid mit neun bis fünfzig Aminosäuren aufweist, die eine Sequenz haben, die innerhalb der Aminosäuresequenz aus Anhang A vorkommt, wobei dieses Peptid in der Lage ist, mit Antikörpern zu reagieren, die mit dem Hauptprotein der Außenmembran der C. trachomatis reagieren.

14. Verfahren gemäß Anspruch 13, bei dem das Peptid mit einem Träger verbunden ist.

15. Verfahren zum Nachweis von Chlamydia trachomatis Antigen oder Antikörpern gegen C. trachomatis in einer biologischen Probe, gekennzeichnet durch die Verwendung eines C. trachomatis MOMP-Polypeptides oder Fragments davon, das durch ein DNA-Konstrukt kodiert ist, das die folgenden operabel verbunden Elemente aufweist:
ein Transkriptionspromotor;
ein DNA-Molekül, das ein C. trachomatis MOMP-Polypeptid kodiert und ein MOMP-Polynukleotid mit einer Länge von wenigstens 27 Basenpaaren der Sequenz aus Anhang A aufweist;
und
ein Transkriptionsterminator, wobei wenigstens eine dieser transkriptionalen- oder translationalen regulativen Sequenzen sich nicht von Chlamydia trachomatis ableitet.

16. Verfahren zum Nachweis von Chlamydia trachomatis Antigen oder Antikörpern gegen C. trachomatis in einer biologischen Probe, gekennzeichnet durch die Verwendung eines C. trachomatis MOMP-Polypeptides oder Fragments davon, das durch ein DNA-Konstrukt kodiert ist, das die folgenden operabel verbunden Elemente aufweist:
ein Transkriptionspromotor;
ein DNA-MOMP-Polynukleotid-Molekül, das wenigstens neun aufeinander folgende Aminosäuren der Aminosäuresequenz des Chlamydia trachomatis MOMP aus Anhang A kodiert, wobei das MOMP-Polynukleotid mit einem zweiten Strukturgen in dem richtigen Leseraster fusioniert ist und sich unter der regulativen Kontrolle des regulierenden Systems des zweiten Strukturgens befindet;
und
ein Transkriptionsterminator, wobei wenigstens eine dieser transkriptionalen regulativen Sequenzen sich nicht von Chlamydia trachomatis ableitet.

## Revendications (Revendications pour l'(les) Etat(s) contractant(s) suivant(s): BE, CH, DE, FR, GB, IT, LI, LU, NL, SE)

1. Construction d'ADN pour l'expression de polypeptides PPME de Chlamydia trachomatis, comprenant les éléments suivants liés de manière fonctionnelle:
un promoteur transcriptionnel; pour un polypeptide
une molécule d'ADN codant de C. trachomatis comprenant un polynucléotide PPME d'au moins 27 paires de base de long de la séquence de l'annexe A;
et
un terminateur transcriptionnel, caractérisé en ce qu'au moins l'une desdites séquences de régulation transcriptionnelles ou traductionnelles n'est pas issue de Chlamydia trachomatis.

2. Construction d'ADN selon la revendication 1, caractérisée en ce que le polynucléotide PPME code sensiblement pour toute la longueur de la protéine principale de la membrane externe.

3. Construction d'ADN selon la revendication 1, caractérisée en ce que le polynucléotide PPME code pour un polypeptide réactif vis-à-vis d'anticorps spécifiques de Chlamydia.

4. Construction d'ADN selon la revendication 1, caractérisée en ce que le polynucléotide PPME code pour un polypeptide réactif vis-à-vis d'anticorps spécifiques de C. trachomatis.

5. Construction d'ADN selon la revendication 1, caractérisée en ce que le polynucléotide PPME code pour un polypeptide réactif vis-à-vis d'anticorps spécifiques d'une sérovar de C. trachomatis.

6. Construction d'ADN pour l'expression de polypeptide PPME de Chlamydia trachomatis, comprenant les éléments suivants liés de manière fonctionnelle:
un promoteur transcriptionnel;
une molécule d'ADN pour le polynucléotide PPME, codant pour au moins neuf acides aminés consécutifs de la séquence d'acides aminés de PPME de Chlamydia trachomatis de l'annexe A, caractérisée en ce que le polynucléotide PPME est fusionné à un deuxième gène de structure en bonne phase de lecture avec lui et sous le contrôle de régulation du système de régulation du deuxième gène de structure; et
un terminateur transcriptionnel, caractérisé en ce qu'au moins l'une desdites séquences de régulation transcriptionnelles n'est pas issue de Chlamydia trachomatis.

7. Construction' d'ADN selon la revendication 6, caractérisée en ce que le deuxième gène de structure code pour un immunogène.

8. Construction d'ADN selon la revendication 6, caractérisée en ce que le deuxième gène de structure est un gène de la β-galactosidase.

9. Molécule polynucléotidique isolée comprenant au moins 27 paires de base de long de la séquence de l'annexe A et codant pour un polypeptide présentant une réactivité immunologique croisée avec la protéine principale de la membrane externe de Chlamydia trachomatis, ledit polynucléotide étant dépourvu d'au moins une région flanquante naturelle.

10. Polynucléotide isolé selon la revendication 9, caractérisé en ce que le polypeptide ainsi codé présente une réactivité immunologique croisée avec de la PPME de sérovar L2.

11. Sonde polynucléotidique isolée pour détecter la présence de Chlamydia dans un échantillon biologique, ladite sonde comprenant un fragment polynucléotidique d'au moins 12 nucléotides de la séquence nucléotidique PPME de C. trachomatis de l'annexe A, laquelle sonde est susceptible de se lier à une séquence d'ADN ou d'ARN caractéristique de Chlamydia.

12. Sonde polynucléotidique isolée selon la revendication 11, caractérisée en ce que la sonde est susceptible de se lier à une séquence d'ADN ou d'ARN caractéristique de C. trachomatis.

13. Sonde polynucléotidique isolée selon la revendication 11, caractérisée en ce que la sonde est susceptible de se lier à une séquence d'ADN ou d'ARN caractéristique d'une sérovar de C. trachomatis.

14. Sonde polynucléotidique selon la revendication 13, caractérisée en ce que ledit fragment polynucléotidique comprend en outre un marqueur détectable.

15. Cellule en culture transformée ou transfectée par une construction d'ADN selon la revendication 1 ou 6.

16. Cellule transformée selon la revendication 15, caractérisée en ce qu'il s'agit de E. coli.

17. Peptide, comprenant de neuf à cinquante acides aminés ayant une séquence se situant dans la séquence d'acides aminés de l'annexe A et lequel peptide est susceptible de réagir avec des anticorps réagissant avec la protéine principale de la membrane externe de C. trachomatis.

18. Peptide selon la revendication 17, pour une utilisation dans un immunodosage pour la détection d'anticorps contre C. trachomatis ou l'antigène de C. trachomatis dans un échantillon biologique.

19. Peptide selon la revendication 17, caractérisé en ce qu'il est conjugué à un agent porteur.

20. Immunodosage de détection de l'antigène de Chlamydia trachomatis ou d'anticorps contre C. trachomatis dans un échantillon biologique, ledit immunodosage étant caractérisé par l'utilisation d'un polypeptide PPME de C. trachomatis ou d'un fragment de celui-ci codé par une construction d'ADN selon la revendication 1 ou 6, où le polypeptide peut être marqué ou non.

## Revendications (Revendications pour l'(les) Etat(s) contractant(s) suivant(s): AT)

1. Procédé d'expression de polypeptides PPME de Chlamydia trachomatis, caractérisé par l'utilisation d'une construction d'ADN comprenant les éléments suivants liés de manière fonctionnelle:
un promoteur transcriptionnel; pour un polypeptide
une molécule d'ADN codant de C. trachomatis comprenant un polynucléotide PPME d'au moins 27 paires de base de long de la séquence de l'annexe A;
et
un terminateur transcriptionnel, caractérisé en ce qu'au moins l'une des séquences de régulation transcriptionnelles ou traductionnelles n'est pas issue de Chlamydia trachomatis.

2. Procédé selon la revendication 1, caractérisé en ce que le polynucléotide PPME code sensiblement pour toute la longueur de la protéine principale de la membrane externe.

3. Procédé selon la revendication 1, caractérisé en ce que le polynucléotide PPME code pour un polypeptide réactif vis-à-vis d'anticorps spécifiques de Chlamydia.

4. Procédé selon la revendication 1, caractérisé en ce que le polynucléotide PPME code pour un polypeptide réactif vis-à-vis d'anticorps spécifiques de C. trachomatis.

5. Procédé selon la revendication 1, caractérisé en ce que le polynucléotide PPME code pour un polypeptide réactif vis-à-vis d'anticorps spécifiques d'une sérovar de C. trachomatis.

6. Procédé pour l'expression de polypeptides PPME de Chlamydia trachomatis, caractérisé par l'utilisation d'une construction d'ADN comprenant les éléments suivants liés de manière fonctionnelle:
un promoteur transcriptionnel;
une molécule d'ADN pour le polynucléotide PPME, codant pour au moins neuf acides aminés consécutifs de la séquence d'acides aminés de PPME de Chlamydia trachomatis de l'annexe A, caractérisée en ce que le polynucléotide PPME est fusionné à un deuxième gène de structure en bonne phase de lecture avec lui et sous le contrôle de régulation du système de régulation du deuxième gène de structure; et
un terminateur transcriptionnel, caractérisé en ce qu'au moins l'une desdites séquences de régulation transcriptionnelles n'est pas issue de Chlamydia trachomatis.

7. Procédé selon la revendication 6, caractérisé en ce que le deuxième gène de structure code pour un immunogène.

8. Procédé selon la revendication 6, caractérisé en ce que le deuxième gène de structure est un gène de la β-galactosidane.

9. Procédé de détection de la présence de Chlamydia trachomatis dans un échantillon biologique, caractérisé par l'utilisation d'une sonde polynucléotidique isolée, ladite sonde comprenant un fragment polynucléotidique d'au moins 12 nucléotides de la séquence nucléotidique PPME de Chlamydia trachomatis de l'annexe A, laquelle sonde est susceptible de se lier à une séquence d'ADN ou d'ARN caractéristique de Chlamydia trachomatis.

10. Procédé selon la revendication 9, caractérisé en ce que la sonde est susceptible de se lier à une séquence d'ADN ou d'ARN caractéristique de C. trachomatis.

11. Procédé selon la revendication 9, caractérisé en ce que la sonde est susceptible de se lier à une séquence d'ADN ou d'ARN caractéristique d'une sérovar de C. trachomatis.

12. Procédé selon la revendication 11, caractérisé en ce que ledit fragment polynucléotidique comprend en outre un marqueur détectable.

13. Procédé de détection d'anticorps contre C. trachomatis ou l'antigène de C. trachomatis dans un échantillon biologique, caractérisé par l'utilisation d'un immunodosage comprenant un peptide comprenant de neuf à cinquante acides aminés ayant une séquence se situant dans la séquence d'acides aminés de l'annexe A et lequel peptide est susceptible de réagir avec des anticorps réagissant avec la protéine principale de la membrane externe de C. trachomatis.

14. Procédé selon la revendication 13, caractérisé en ce que le peptide est conjugué à un agent porteur.

15. Procédé de détection de l'antigène de Chlamydia trachomatis ou d'anticorps contre C. trachomatis dans un échantillon biologique, caractérisé par l'utilisation d'un polypeptide PPME de C. trachomatis ou d'un fragment de celui-ci codé par une construction d'ADN, comprenant les éléments suivants liés de manière fonctionnelle:
un promoteur transcriptionnel;
une molécule d'ADN codant pour un polypeptide PPME de C. trachomatis comprenant un polynucléotide PPME d'au moins 27 paires de base de long de la séquence de l'annexe A;
et
un terminateur transcriptionnel, caractérisé en ce qu'au moins l'une desdites séquences de régulation transcriptionnelles ou traductionnelles n'est pas issue de Chlamydia trachomatis.

16. Procédé de détection de l'antigène de Chlamydia trachomatis ou d'anticorps contre C. trachomatis dans un échantillon biologique, caractérisé par l'utilisation d'un polypeptide PPME de C. trachomatis ou d'un fragment de celui-ci codé par une construction d'ADN, comprenant les éléments suivants liés de manière fonctionnelle:
un promoteur transcriptionnel;
une molécule d'ADN pour le polynucléotide PPME, codant pour au moins neuf acides aminés consécutifs de la séquence d'acides aminés de PPME de Chlamydia trachomatis de l'annexe A, caractérisée en ce que le polynucléotide PPME est fusionné à un deuxième gène de structure en bonne phase de lecture avec lui et sous le contrôle de régulation du système de régulation du deuxième gène de structure; et
un terminateur transcriptionnel, caractérisé en ce qu'au moins l'une desdites séquences de régulation transcriptionnelles n'est pas issue de Chlamydia trachomatis.
